# FASCICULE DE BREVET EUROPEEN

(11) **EP 1 357 896 B1**
(45) Date de publication et mention de la délivrance du brevet: **12.03.2008**
(21) Numéro de dépôt: 02703650.8
(22) Date de dépôt: 29.01.2002
(51) Int. Cl.: A61K 9/50, A61F 2/02

(54) **MEMBRANE POUR CHAMBRE D'ENCAPSULATION DE CELLULES PRODUISANT AU MOINS UNE SUBSTANCE BIOLOGIQUEMENT ACTIVE ET ORGANE BIO-ARTIFICIEL COMPRENANT UNE TELLE MEMBRANE.**
MEMBRAN FÜR EINE EINKAPSELUNGSKAMMER VON ZELLEN DIE ZUMINDESTENS EINEN WIRKSTOFF ERZEUGEN UND BIOARTIFIZIELLES ORGAN ENTHALTEND DIESE MEMBRAN
MEMBRANE FOR ENCAPSULATION CHAMBER OF CELLS PRODUCING AT LEAST A BIOLOGICALLY ACTIVE SUBSTANCE AND BIOARTIFICIAL ORGAN COMPRISING SAME

(30) Priorité: 30.01.2001 FR 0101248
(43) Date de publication de la demande: 05.11.2003
(73) Titulaire: Association pour les transferts de technologie Du Mans, 72000 Le Mans (FR); UNIVERSITE CATHOLIQUE DE LOUVAIN, 1348 Louvain la Neuve (BE); Centre Europeen D'Etude Du Diabete (Ceed), 67000 Strassbourg (FR)
(72) Inventeur: LEGEAY, Gilbert, F-72650 Saint Saturnin (FR); BERTRAND, Patrick, B-1348 Louvain-la-Neuve (BE); BELCOURT, Alain, F-67200 Strasbourg (FR); KESSLER, Laurence, F-67205 Oberhausbergen (FR)
(74) Mandataire: Catherine, Alain
(86) Numéro de dépôt international: PCT/FR2002/000347
(87) Numéro de publication internationale: WO 2002/060409

(56) Documents cités:
- WO-A-90/04609
- WO-A-93/16687
- WO-A-94/18906
- WO-A-95/26714
- WO-A-97/30778
- WO-A-98/13405
- WO-A-98/28026

## Description

La présente invention concerne une membrane pour chambre d'encapsulation de cellules produisant au moins une substance biologiquement active d'intérêt ayant des caractéristiques de perméabilité et de biocompatibilité améliorées. L'invention est également relative à une chambre d'encapsulation de cellules contenant au moins une telle membrane ainsi qu'à un organe bio-artificiel comprenant au moins une chambre d'encapsulation de cellules.

Le traitement de pathologies nécessitant un apport continu à l'organisme en substances biologiquement actives a rendu nécessaire la mise au point de dispositifs capables de libérer ces substances biologiquement actives de manière efficace et qui puissent être implantés chez le patient, parfois pendant de longues périodes de temps. De tels dispositifs sont, par exemple, des organes bio-artificiels qui renferment des cellules produisant une ou plusieurs substances biologiquement actives d'intérêt. Les cellules contenues dans un organe bio-artificiel sont confinées dans des espaces internes ou chambres d'encapsulation délimitées par au moins une membrane semi-perméable. Une telle membrane semi-perméable doit laisser passer les substances biologiquement actives d'intérêt qui doivent être accessibles aux cellules cibles visées dans le corps du patient, tout en étant imperméable aux cellules du patient, plus particulièrement les cellules du système immunitaire, ainsi qu'aux anticorps et autres substances toxiques.

De tels organes bio-artificiels comprenant une membrane semi-perméable sont par exemple décrits dans la demande PCT N°WO 94/18 906 publiée le 1er Septembre 1994 ou encore dans les brevets US N°4,323,457 délivré le 6 Avril 1982 et US N°6,023,009 délivré le 8 Février 2000.

Différents types de membranes semi-perméables pour organes bio-artificiels ont été décrits dans l'état de la technique, tel que par exemple des membranes de polyacrylonitrile, de polyéthersulfone, de résine acrylique, d'acétate de cellulose, de nitrate de cellulose, de polyamide, ou encore d'hydroxypropylméthylcellulose (HPMC).

L'utilisation d'une membrane de polycarbonate a également été envisagée, parmi de nombreux autres types de membranes, dans la demande PCT N°WO 94/18.906. Toutefois, le mode de réalisation préférentiel décrit dans cette demande PCT consiste en l'utilisation d'une membrane poreuse d'un copolymère acrylique d'une porosité de 50,000 à 80.000 Daltons, telle que la membrane XM fabriquée par la Division Amicon de la Société GRACE & Cie.

Les membranes semi-perméables pour organes bio-artificiels préconisées dans l'état de la technique présentent certains inconvénients.

Ainsi, les membranes semi-perméables en polyacrylonitrile, par exemple les membranes du type « AN69 », possèdent naturellement des pores ayant un seuil de coupure d'environ 60.000 Daltons et sont généralement utilisées dans des dispositifs d'ultrafiltration. Les membranes « AN69 » sont des membranes de polyacrylonitrileméthallylsulfonate de sodium, commercialisées par la Société HOSPAL.

Ces membranes se détériorent rapidement si elles ne sont pas impérativement manipulées dans de l'eau ou dans une solution aqueuse.

Les difficultés de manipulation des membranes de polyacrylonitrile ci-dessus se retrouvent de manière générale pour toutes les membranes semi-perméables à base d'hydrogel.

A la connaissance du demandeur, il n'a pas été décrit dans l'état de la technique de membrane semi-perméable qui combine les propriétés suivantes:
- une bonne résistance à la manipulation sans détérioration lorsqu'elles sont manipulées en dehors d'une solution aqueuse, par exemple à l'air ;
- d'excellentes propriétés de perméabilité aux substances biologiquement actives produites à l'intérieur des organes bio-artificiels ainsi qu'une bonne perméabilité aux divers composés nutriments de l'organisme nécessaires à la viabilité des cellules contenues dans l'organe bio-artificiel;
- une imperméabilité aux molécules de grande taille produites par l'organisme, plus particulièrement les immunoglobulines, ainsi qu'aux cellules du système immunitaire, afin de protéger les cellules de l'organe bio-artificiel d'une réaction immunitaire de l'organisme hôte pouvant conduire au rejet de l'implant.
- une capacité réduite à adsorber à sa surface les protéines présentes dans l'organisme au lieu d'implantation de l'organe bio-artificiel;
- une faible adhésion des cellules à sa surface;
- une rapidité des échanges de molécules passant à travers la membrane, assurant une biodisponibilité rapide des substances biologiquement actives d'intérêt produites par l'organe bio-artificiel ainsi qu'une bonne accessibilité des nutriments de l'organisme aux cellules contenues dans cet organe artificiel, y compris des molécules produites par l'hôte pouvant constituer des signaux nécessaires à la régulation de la synthèse des substances biologiquement actives par les cellules contenues dans l'organe bio-artificiel.

Une membrane semi-perméable pour organe bio-artificiel possédant l'ensemble des propriétés recherchées énumérées ci-dessus est fournie selon l'invention.

L'invention a donc pour objet une membrane semi-perméable pour chambre d'encapsulation de cellules produisant au moins une substance biologiquement active d'intérêt, caractérisée en ce qu'elle consiste en un film biocompatible de polycarbonate poreux modifié en surface par création de sites polaires et recouvert sans greffage chimique par une couche d'au moins un polymère hydrophile.

Il a en effet été montré selon l'invention qu'une membrane de polycarbonate poreux dont l'énergie de surface a été accrue par création de sites polaires et recouvert sans greffage chimique par une couche d'au moins un polymère hydrophile possédait d'excellentes propriétés de résistance mécanique. Ces excellentes propriétés mécaniques rendent la membrane semi-perméable de l'invention aisément manipulable, sans précaution particulière. De plus, ces propriétés de résistance mécanique confèrent à la membrane semi-perméable des caractéristiques de résistance aux diverses contraintes subies par un implant d'organe bio-artificiel, lorsque ce dernier est introduit pendant une longue période de temps dans le corps d'un organisme hôte.

La membrane semi-perméable selon l'invention, du fait qu'elle possède à sa surface une couche d'au moins un polymère hydrophile, possède une capacité réduite d'adsorption des protéines présentes dans le milieu environnant ainsi qu'une capacité réduite d'adhésion aux cellules de l'organisme hôte présentes au lieu d'implantation. En particulier, les inventeurs ont montré, par des mesures de spectroscopie photo-électronique à rayons X (XPS pour « X-ray photoelectron spectroscopy ») et de TOF-SIMS (« Time of Flight Secondary ion Mass Spectroscopy ») que des protéines comme l'albumine et l'insuline sont faiblement adsorbées à la surface de la membrane semi-perméable de l'invention, d'une part, et que, d'autre part, ces protéines n'étaient pas stablement adsorbées et pouvaient en conséquence se désorber spontanément de la surface de la membrane. En outre, le demandeur a montré qu'une valeur plateau d'adsorption de ces protéines était atteinte pour une faible valeur de concentration protéique, de l'ordre du mg/ml, ce qui semble indiquer que les protéines sont adsorbées tout au plus sous forme d'une mono-couche.

La faible adhésivité des protéines à la surface de la membrane semi-perméable de l'invention permet de réduire considérablement la détérioration des propriétés de perméabilité du fait de l'absence d'obstruction des pores de cette membrane, contrairement à ce qui est observé avec des membranes semi-perméables de l'état de la technique.

De plus, il va sans dire que les caractéristiques de biocompatibilité qui dépendent principalement des interactions entre les protéines du milieu environnant et la surface de la membrane semi-perméable sont considérablement améliorées.

En particulier, une membrane semi-perméable de polycarbonate poreux non recouvert de polymère hydrophile ne possède pas les propriétés de biocompatibilité ni de maintien des caractéristiques de perméabilité à long terme de la membrane selon l'invention.

En outre, la membrane semi-perméable selon l'invention est caractérisée en ce que la couche d'au moins un polymère hydrophile est maintenue durablement à la surface du film de polycarbonate grâce à la création de sites polaires qui accroît l'énergie de surface du film de polycarbonate et permet ainsi l'adhérence de la couche d'au moins un polymère hydrophile par l'intermédiaire de nombreuses liaisons faibles, telles que les liaisons hydrogène, des liaisons ioniques ou encore par des forces de Van der Waals.

La couche d'au moins un polymère hydrophile adhère sans greffage chimique à la surface du film biocompatible de polycarbonate par la formation de liaisons non-covalentes entre les sites polaires créés à la surface du film de polycarbonate et les groupements hydrophiles du polymère, sans liaison covalente entre le polymère hydrophile et le film de polycarbonate.

En général, le greffage de composés monomères ou polymères hydrophiles à la surface d'un support n'est jamais complet et peut en conséquence conduire à l'obtention de produits finis comportant des monomères libres non greffés, lesquels, dans le cas par exemple de composés monomères halogénés, peuvent entraîner des réactions d'irritation après implantation dans le corps d'un organisme hôte. De tels inconvénients ne sont donc pas rencontrés avec la membrane semi-perméable de l'invention.

WO-A-98/28026 et WO-A-90/04609 décrivent des revêtements obtenus par greffage chimique.

La présence de la couche d'au moins un polymère hydrophile à la surface de la membrane semi-perméable selon l'invention permet l'obtention d'une vitesse optimale d'échanges de molécules traversant la membrane du fait que ces molécules ne sont pas adsorbées, même de manière transitoire, sur la surface de la membrane semi-perméable. Les substances biologiquement actives produites par les cellules de l'organe bio-artificiel sont rapidement accessibles aux cellules cibles de l'organisme hôte. De plus, l'activation du flux des échanges entre l'extérieur et l'intérieur de l'organe bio-artificiel permet également une accessibilité accrue des nutriments aux cellules contenues dans l'implant, favorisant leur survie à long terme et donc le fonctionnement à long terme de l'organe bio-artificiel. La présence de la couche d'au moins un polymère hydrophile à la surface d'une membrane semi-perméable confère donc à cette membrane d'excellentes propriétés hydrodynamiques encore jamais atteintes avec les membranes de l'état de la technique.

La création de sites polaires à la surface du film biocompatible de polycarbonate correspond principalement à l'augmentation de la proportion de groupements carbonyle, hydroxy ou amine, et des radicaux libres. Les radicaux libres se recombinent entre eux, ou avec l'oxygène de l'air, créant ainsi des sites polaires.

De préférence, les sites polaires présents à la surface du film biocompatible de polycarbonate constitutif de la membrane semi-perméable de l'invention comprennent les sites suivants:
CH₃O, C₂H₃O, C₃H₃O, C₃H₇O, O, OH, C₂OH, C₈H₅O, NH₄⁺, C₂H₈N⁺, R-OH (alcool), (R)₃-NH (amine) et R-CO-NH (amide), dans lesquels le substituant R représente un radical constitutif du polymère de polycarbonate du film de polycarbonate poreux.

De manière tout à fait préférée, la membrane semi-perméable selon l'invention est caractérisée en ce que la taille des pores du film de polycarbonate est comprise entre 5 et 30 nanomètres, de préférence entre 5 et 15 nanomètres.

La membrane semi-perméable selon l'invention est aussi caractérisée en ce qu'elle possède un seuil de coupure compris entre 10.000 et 50.000 Daltons, de préférence entre 10.000 et 30.000 Daltons, et de manière tout à fait préférée entre 10.000 et 15.000 Daltons.

Selon encore une autre caractéristique, la membrane semi-perméable selon l'invention possède une densité de pores comprise entre 10⁹ et 10¹¹ pores/cm².

Les caractéristiques à la fois de taille des pores et de leur densité impliquent que le pourcentage de surface ouverte de la membrane semi-perméable selon l'invention est très faible et ne provoque pas de diminution significative des propriétés mécaniques de résistance qui sont observées pour un film de polycarbonate non poreux.

Avantageusement, la membrane semi-perméable de l'invention possède une épaisseur comprise entre 5 µm et 25 µm, de préférence entre 10 µm et 20 µm.

Selon une autre caractéristique avantageuse de la membrane semi-perméable de l'invention, la couche d'au moins un polymère hydrophile possède une épaisseur comprise entre 10 et 100 nanomètres, préférentiellement entre 10 et 50 nanomètres et de manière tout à fait préférée entre 10 et 30 nanomètres.

Selon un premier mode de réalisation, la couche d'au moins un polymère hydrophile peut recouvrir les deux faces du film biocompatible de polycarbonate.

Dans un second mode particulier de réalisation, la couche d'au moins un polymère hydrophile ne recouvre qu'une seule des deux faces du film biocompatible de polycarbonate, préférentiellement la face du film de polycarbonate qui est située du côté externe de l'organe bio-artificiel et qui est en contact avec le milieu environnant le lieu d'implantation de cet organe.

Le film biocompatible de polycarbonate constitutif d'une membrane semi-perméable de l'invention peut par exemple être fabriqué conformément à l'enseignement du brevet américain n°US 4,956,219 délivré le 11 Septembre 1990.

Ce brevet américain décrit un film de polycarbonate dont les pores ont été formées par bombardement électronique ou par bombardement d'ions lourds, avant une étape chimique d'érosion. La densité des ions lourds bombardés à la surface du film de polycarbonate détermine la densité des pores tandis que le temps de traitement d'érosion chimique détermine la taille des pores créées. La mise en oeuvre d'une telle technique permet l'obtention de film de polycarbonate biocompatible poreux possédant une grande homogénéité dans la distribution des pores ainsi qu'une grande homogénéité dans la taille des pores. Ces caractéristiques confèrent à la membrane semi-perméable de l'invention une excellente reproductibilité dans la valeur du seuil de coupure ainsi que dans le volume des échanges des fluides à travers la membrane.

Constitue un polymère hydrophile au sens de l'invention, un polymère, lequel, après application sur un film de polycarbonate poreux présente une valeur d'angle inférieure à 25°; de préférence inférieure à 22° après mesure selon le test de la « goutte sessile » décrit à l'exemple 2.

Préférentiellement, le polymère hydrophile est soluble dans l'eau. En effet, du fait de l'implantation de l'organe bio-artificiel dans le corps d'un organisme hôte, l'utilisation de solvants organiques est exclu car leur élimination totale est difficile, et leur présence, même en faibles quantités, n'est pas compatible avec une utilisation thérapeutique ou chirurgicale chez l'homme ou l'animal.

De préférence, le matériau polymère hydrophile est choisi parmi les polymères hydrophiles suivants :
- les celluloses et leurs dérivés, tels que l'hydroxypropyl méthylcellulose (HPMC), par exemple la HPMC E4M commercialisée par la Société DOW CHEMICALS, ou celle dénommée Aqualon commercialisée par la Société Herculès, ou encore la carboxyméthylcellulose commercialisée par la Société DOW CHEMICALS;
- les polyacrylamides et leurs copolymères, tels que ceux commercialisés par la Société SIGMA (UPSALA, Suède):

- la polyvinylpyrrolidone (PVP) et ses copolymères, tels que ceux commercialisés par la Société BASF/Laserson, comme le Kollidon;
- les copolymères de l'acétate de vinyle, tel que le copolymère de polyacétate de vinyle et d'alcool polyvinylique commercialisé sous le nom de Mowiol par la Société HOECHST/CLARIANT;
- les polyéthylène glycols, tels que ceux commercialisés par la Société SIGMA;
- les propylène glycols ;
- les poly(méth)acrylates hydrophiles, tels que ceux commercialisés par les Sociétés DEGALAN ou DEGUSSA;
- les polyosides;
- les chitosans, tels que ceux commercialisés par la Société SIGMA.

On entend par polymère hydrophile selon l'invention, aussi bien un matériau polymère constitué de l'un des polymères hydrophiles tels que définis ci-dessus qu'un mélange de plusieurs des polymères hydrophiles ci-dessus, en général un mélange de deux ou de trois des polymères hydrophiles ci-dessus.

L'invention a également pour objet une chambre d'encapsulation de cellules produisant au moins une substance biologiquement active d'intérêt caractérisée en ce qu'elle comprend au moins une membrane semi-perméable telle que définie ci-dessus.

Une chambre d'encapsulation selon l'invention peut avoir les caractéristiques de toute chambre d'encapsulation de cellules connue de l'état de la technique, et plus particulièrement des chambres d'encapsulation de cellules qui sont constitutives d'organes bio-artificiels décrits dans l'état de la technique.

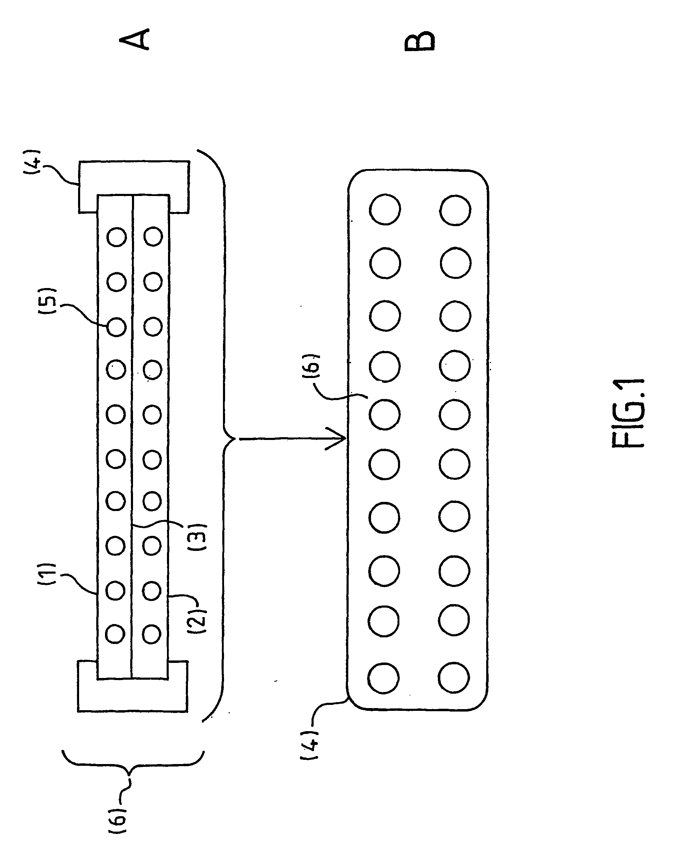

A titre illustratif, mais non limitatif, d'un mode de réalisation d'une chambre d'encapsulation de cellules selon l'invention, une telle chambre d'encapsulation est telle qu'illustrée à la figure 1A.

Selon ce mode de réalisation particulier, la chambre d'encapsulation est de forme cylindrique et comprend un support (4) sur lequel vient se solidariser le bord externe d'une membrane semi-perméable telle que définie ci-dessus. La chambre d'encapsulation comprend au moins deux membranes semi-perméables, respectivement une membrane semi-perméable supérieure (1) et une membrane semi-perméable inférieure (2) lesquelles, en combinaison avec les bords du support externe (4) délimitant l'enceinte de la chambre d'encapsulation dans laquelle sont contenues des cellules produisant au moins une substance biologiquement active d'intérêt.

La chambre d'encapsulation selon l'invention peut également comprendre une troisième membrane semi-perméable (3) conforme à l'invention de telle manière à ce que ladite chambre d'encapsulation comprenne deux enceintes closes distinctes contenant chacune des cellules produisant une substance biologiquement active d'intérêt.

L'invention a donc encore pour objet une chambre d'encapsulation de cellules produisant au moins une substance biologiquement active d'intérêt caractérisée en ce qu'elle comprend deux membranes semi-perméables conformes à l'invention, respectivement inférieures et supérieures, dont les bords externes sont solidaires d'un support, les deux membranes délimitant un espace susceptible de contenir les cellules produisant au moins une substance biologiquement active d'intérêt.

La chambre d'encapsulation selon l'invention peut être de forme circulaire.

Les cellules produisant au moins une substance biologiquement active d'intérêt peuvent être, par exemple, des cellules des îlots de Langherans, qui produisent de l'insuline lorsque la chambre d'encapsulation est destinée à la fabrication d'un pancréas bio-artificiel.

Les cellules peuvent aussi être des cellules hépatiques lorsque la chambre d'encapsulation est destinée à la fabrication d'un foie bio-artificiel.

Dans un mode de réalisation particulier, les cellules sont transfectées ou transformées par au moins un acide nucléique permettant l'expression d'une substance biologiquement active d'intérêt. Parmi les substances biologiquement actives d'intérêt, on peut citer, à titre illustratif, l'insuline, les cytokines, les hormones peptidiques, l'hormone de croissance et la calcitonine.

De manière générale, on entend par « substance biologiquement active » au sens de l'invention, une substance qui est libérée ou sécrétée par la cellule qui la produit et exerce son effet sur une cellule cible ou sur une molécule cible dans l'organisme hôte, comme par exemple un neurotransmetteur, une hormone, un facteur de croissance ou une cytokine.

Une grande diversité de cellules peut être utilisée, incluant des lignées cellulaires immortalisées comme des cultures primaires de cellules en division.

Les cellules peuvent être par exemple des myoblastes, qui sont des cellules précurseurs des cellules musculaires dérivées des populations de cellules souches du mésoderme, et qui peuvent être facilement transformées par un acide nucléique permettant l'expression de la substance biologiquement active d'intérêt. L'homme du métier pourra avantageusement se référer par exemple aux demandes PCT publiées sous les numéros WO 94/02129, WO 93/03768 et WO 90/15863.

Les cellules peuvent aussi être des cellules bêta des îlots de Langherans du pancréas ou encore des hépatocytes, de préférence d'origine humaine.

De préférence, les cellules contenues dans une chambre d'encapsulation selon l'invention sont incluses dans une matrice, telle qu'une matrice de collagène de type IV, le cas échéant en association avec de la laminine, de l'entactine et de l'héparane sulfate comme la matrice commercialisée sous le nom de Matrigel.

Les cellules contenues dans une chambre d'encapsulation conforme à l'invention peuvent, de manière générale, être incluses dans une matrice composée de tout produit ou combinaison de produits permettant l'immobilisation de ces cellules sous une forme viable.

Les cellules produisant au moins une substance biologiquement active d'intérêt peuvent être également encapsulées dans une matrice d'alginate.

Selon un autre aspect, l'invention est également relative à un organe bio-artificiel caractérisé en ce qu'il comprend une chambre d'encapsulation ou une pluralité de chambres d'encapsulation telles que définies ci-dessus.

Les caractéristiques d'un organe bio-artificiel selon l'invention peuvent être de toute nature connue en soi dans l'état de la technique.

Pour la réalisation d'un organe bio-artificiel de l'invention, dont la caractéristique essentielle est de comprendre au moins une chambre d'encapsulation de cellules dotées d'une membrane semi-perméable telle que définie dans la présente description, l'homme du métier pourra avantageusement se référer aux brevets US N° 5,981,211 délivré le 9 Novembre 1999. US 4.578.191 délivré le 25 Mars 1986. US 5.837.234 délivré le 17 Novembre 1998, US 6,023,009 délivré le 8 Février 2000, US 5,605,835 délivré le 25 Février 1997 et US N°4,323,457 délivré le 6 Avril 1982.

Dans un mode de réalisation particulier de l'organe bio-artificiel selon l'invention, cet organe bio-artificiel comprend une pluralité de chambres d'encapsulation de cellules, comme cela est illustré à la figure 1B. Un tel organe bio-artificiel comprend un support (4) comprenant une pluralité de chambres d'encapsulation (6). Le support (4) peut être constitué par exemple en silicone.

Selon un mode de réalisation particulier de l'invention, l'organe bio-artificiel est un pancréas bioartificiel contenant des cellules des îlots de Langherans, de préférence encapsulées au sein d'une matrice.

Selon un second mode particulier de réalisation de l'invention, l'organe bio-artificiel est un foie artificiel contenant des cellules hépatiques.

A titre illustratif, un organe bio-artificiel selon l'invention peut être implanté de manière intra-péritonéale ou encore au-dessus de la capsule rénale.

Un autre objet de l'invention consiste en un procédé d'obtention d'une membrane semi-perméable pour chambres d'encapsulation de cellules produisant au moins une substance biologiquement active d'intérêt, caractérisé en ce qu'il comprend les étapes suivantes:
a) création de sites polaires à la surface d'un film biocompatible de polycarbonate poreux;
b) trempage du film de polycarbonate ainsi traité dans une solution acqueuse d'au moins un polymère hydrophile sans greffage chimique; et
c) séchage.

De manière préférentielle, la création de sites polaires à la surface du film biocompatible de polycarbonate est réalisée par un traitement par plasma, par décharge couronne ou encore par décharge électromagnétique à pression atmosphérique ou sous vide.

De manière préférentielle, le support est traité par un plasma radiofréquence d'argon. Il peut être traité à une puissance d'émission du réacteur à plasma comprise entre 3 et 10 watts par litre de capacité du réacteur, durant entre environ 1 et 20 minutes. Le traitement peut aussi être effectué par un plasma micro-onde, à la même puissance, mais durant 5 secondes à 20 minutes.

Ue manière préférée, le traitement par plasma est effectué sous vide.

Pour la mise en oeuvre d'un procédé de traitement par plasma, l'homme du métier pourra avantageusement se référer à l'ouvrage de André Ricard intitulé « Plasmas réactifs » et publié aux éditions SVF en 1995.

Le traitement peut aussi être effectué par décharge couronne. La tension de traitement est avantageusement comprise entre 50 et 500 volts, l'intensité étant variable selon le dispositif de traitement et les supports traités. La durée du traitement est de l'ordre de quelques dixièmes de seconde, préférentiellement compris entre 0,1 et 1 seconde. En cas de traitement en continu, la durée d'exposition est telle que le matériau à traiter passe à travers le dispositif de traitement à une vitesse de quelques centimètres à plusieurs décimètres par seconde.

En outre, le film biocompatible de polycarbonate peut être traité à plusieurs reprises afin d'augmenter l'efficacité du traitement.

Le traitement par décharge couronne peut être effectué à l'aide de dispositifs à électrodes parallèles en vis-à-vis, à électrodes parallèles côte à côte (arc électrode d'environ 5 mm de hauteur), ou à arc soufflé (électrodes parallèles côte à côte avec courant gazeux entre elles, créant ainsi un arc électrique d'environ 10 cm de hauteur).

Pour la réalisation d'un procédé de traitement par décharge couronne ou par décharge électromagnétique, l'homme du métier pourra avantageusement se référer à l'ouvrage de André RICHARD (1995) cité ci-dessus.

De manière tout à fait préférée, la création de sites polaires est réalisée par une étape de traitement par plasma d'argon effectuée à la puissance de 50 watts pendant dix minutes. Dans ce mode de réalisation particulier, il a été observé à la surface du film biocompatible de polycarbonate, par mesure de spectrométrie de masse des ions secondaires, la composition suivante, en sites polaires quantifiée par l'intensité des ions secondaires détectés aux rapports masse/charge (m/z) suivants:
- en mode positif: 31 (CH₃O), 43 (C₂H₃O), 55 (C₃H₃O), 59 (C₃H₇O), 18(NH₄⁺) et 46 (C₂H₈N⁺); et
- dans le mode négatif : 16(0), 17(OH), 41(C₂OH), 117 (C₈H₅O); Grâce au traitement par plasma du film biocompatible de polycarbonate, on obtient une oxydation stable dans le temps, notamment par la création de groupes oxydants tels que des groupements alcool, acide et carbonyle ce qui accroît l'hydrophilicité de la surface du film de polycarbonate et donc également son énergie de surface. Par exemple, l'indice de mouillage correspondant à la valeur de l'angle (thêta) pris au point de raccordement d'une goutte de liquide avec la surface du film de polycarbonate sur laquelle elle est déposée passe de 74° environ avant traitement par plasma (Es=41 mJ.m⁻²) à 29°.

Il a été montré selon l'invention que le recouvrement du film biocompatible de polycarbonate, après création de sites polaires, par une couche d'au moins un polymère hydrophile augmentait considérablement ces propriétés d'hydrophilicité, puisque l'indice de mouillage observé après recouvrement par la PVP est d'environ 22°, à comparer avec la valeur d'indice de mouillage de 74° observée pour le film de polycarbonate avant traitement.

L'étape b) de recouvrement du film de polycarbonate après création des sites polaires par une couche d'au moins un polymère hydrophile peut être réalisée par trempage.

Quel que soit le type de polymère hydrophile utilisé, la quantité de ce polymère, en poids total de la solution, est de préférence ajustée de manière à obtenir une solution aqueuse de polymère hydrophile ayant une viscosité comprise entre 1 et 10 centipoises.

Par exemple, une valeur de viscosité de l'ordre de 5 à 10 centipoises (cPs) est obtenue pour une concentration de 1% en poids de PVP (Kollidon K90 commercialisé par la Société BASF) pour une concentration de 0,2% en poids de HPMC (E4M commercialisée par DOW CHEMICALS). Les mesures de viscosité sont réalisées à l'aide d'une aiguille de type DIN 30D, à température ambiante et pour une vitesse de rotation de 300 à 500 t/min.

A titre illustratif, lorsque le polymère hydrophile est de l'hydroxypropyl méthylcellulose (HPMC), de la polyvinylpyrrolidone (PVP) ou encore un mélange de ces deux polymères, le pourcentage en poids du polymère hydrophile, par rapport au poids total de la solution aqueuse de polymère, est avantageusement compris entre 0,1% et 1%. La durée de l'étape de trempage du film de polycarbonate dans une solution de polymère hydrophile est ajustée de telle manière à obtenir une couche de polymère d'une épaisseur comprise entre 10 et 100 nanomètres.

De manière tout à fait préférée, la durée de l'étape du trempage est comprise entre 5 secondes et 10 minutes.

Avantageusement, l'étape de trempage a lieu dans une solution aqueuse de polymère hydrophile à une température comprise entre 15°C et 25°C de préférence à température du laboratoire.

L'étape de séchage c) peut être réalisée par tout moyen connu de l'état de la technique.

Préférentiellement, l'ensemble des étapes du procédé d'obtention d'une membrane semi-perméable selon l'invention est réalisée dans des conditions d'asepsie et en utilisant des matériaux stériles et si possible apyrogènes.

Avantageusement, le procédé selon l'invention comprend une étape supplémentaire de stérilisation de la membrane semi-perméable, qui peut être réalisée indifféremment à froid ou à chaud selon des techniques bien connues de l'homme du métier.

A titre illustratif, l'étape de stérilisation peut être réalisée à l'aide d'une autoclave, par exemple à la température de 121 °C pendant 20 minutes sans entraîner d'altération significative des caractéristiques avantageuses de la membrane semi-perméable.

La membrane semi-perméable peut être conservée stérilement avant usage, par exemple dans une solution saline physiologique telle qu'une solution à 0,9% en poids de chlorure de sodium.

Selon un autre aspect, la membrane semi-perméable de l'invention peut être conservée à sec, préférentiellement à une température d'environ 4°C.

L'invention est en outre illustrée, sans pour autant être limitée, par les figures et les exemples suivants.

### FIGURES

La figure **1** illustre une chambre d'encapsulation de cellules produisant une substance biologiquement active selon l'invention ainsi qu'un organe bio-artificiel comprenant une pluralité de chambres d'encapsulation.

La figure **1A** illustre une coupe transversale d'une chambre d'encapsulation.

La figure **1B** illustre une vue du dessus d'un organe bio-artificiel comprenant 20 chambres d'encapsulation selon l'invention.

### EXEMPLES

### EXEMPLE 1 - Fabrication d'une membrane semi-perméable selon l'invention.

Pour la fabrication d'une membrane semi-perméable selon l'invention, on a utilisé des films biocompatibles de polycarbonate de différentes caractéristiques:
- épaisseur de 10, 16 et 20µm;
- densité de pores de 2.10⁶, 4,5 10⁸, 2.10⁹, 4.10⁹ et 8 10⁹ pores/cm²;
- diamètre de pore 20 à 2000 nanomètres.

Le film biocompatible de polycarbonate a été traité par plasma d'argon dans une enceinte de volume de 20 litres à la puissance de 50 watts pendant 10 minutes à la pression d'environ 1 mbar et à température proche de la température ambiante. La décharge est du type capacitif, à la fréquence de 13,56 Mhz.

Après création de sites polaires à la surface du film de polycarbonate par le traitement par plasma, le film de polycarbonate est soumis à un trempage pendant 1 minute dans une solution aqueuse à 1% en poids de polyvinylpyrrolidone de grade pharmaceutique (Kollidon K90) puis est séché en étuve ventilée pendant 1 heure à une température comprise entre 40°C et 60°C.

### EXEMPLE 2 - Etude des indices de mouillage des membranes semi-perméables hydrophiles selon l'invention.

### Matériel et méthodes.

### 1. Mesure du mouillage

L'indice de mouillage est donné par la valeur de l'angle (thêta) pris au point de raccordement d'une goutte de liquide avec la surface du support sur lequel elle est déposée. Le chiffre correspondant à cet angle déterminé d'un côté par la droite correspondant à la surface de l'objet, et de l'autre côté, par la tangente à la goutte au point de raccordement goutte/surface du support.

Pour une surface hydrophile : la goutte est plate : l'angle est faible ;

Pour une surface hydrophobe : la goutte ressemble à une bille : l'angle est élevé.

### Origine de l'eau

L'eau utilisée pour les mesures est :
- soit déminéralisée par des résines échangeuses d'ions ;
- soit distillée fraîchement ;
- soit d'origine commerciale : préparation injectable (ppi), eau pure.

Cette eau doit être conservée dans des récipients fermés en quartz et à l'abri de la lumière et de la chaleur.

### Dépôt de la goutte

Le volume de la goutte est de quelques microlitres. Dans ces conditions, le poids de la goutte est faible, de sorte que la goutte ne se déforme pas de façon importante sous l'effet de la gravité. Cette goutte peut être produite soit avec une micropipette (type Pasteur), soit avec une microseringue.

### Appareil utilisé

La taille de la goutte ne permet pas de mesurer directement l'angle de contact. Il est nécessaire d'utiliser un appareil optique. Sont proposés :
- un appareil photographique avec objectif macro ;
- un système de projection sur un écran disposant d'un système de graduation des angles ;
- un goniomètre à lunette grossissante : on trouve sur le marché des équipements tels que ceux commercialisés par la Société Ramé-Hart aux Etats-Unis, par la Société Kruss en Allemagne ou par la Société Kyowa au Japon ;
- un goniomètre avec caméra et informatique commercialisé par la Société Kruss ou par la Société GBX Instruments en France (Digidrop).

Il est souhaitable que l'équipement soit localisé dans un local à une température constante, proche de 20°C.

Les mesures présentées dans les exemples ci-après sont faites avec l'équipement Digidrop, selon la technique détaillée ci-dessous.

La goutte est formée à l'extrémité de la seringue (ou de la micropipette), puis le support est rapproché lentement de la goutte (goutte sessile). L'image de la goutte et de la surface apparaît immédiatement sur l'écran : le travail de mesure de l'angle se fait sur cette image.

La mesure est faite soit automatiquement, soit de façon manuelle en pointant avec la souris de l'ordinateur les deux points de raccordement goutte/surface ainsi que le sommet de la goutte : l'ordinateur calcule automatiquement la valeur de l'angle de contact. Il est préférable de réaliser la mesure de façon manuelle, des problèmes de reflets venant parfois perturber complètement la lecture de l'image par la caméra lorsque celle-ci travaille en mode automatique.

La mesure est effectuée environ 5 secondes après le dépôt de la goutte. En effet, dans le cas de certaines surfaces hydrophiles, un étalement progressif au cours du temps peut être constaté. Par ailleurs, une évaporation de l'eau a lieu, ce qui n'est cependant pas perceptible dans les 5 secondes après le dépôt.

Pour chaque surface, un minimum de trois mesures est réalisé et la valeur moyenne est calculée ainsi que la valeur de l'écart type.

Les images et les valeurs des angles sont enregistrées par l'ordinateur.

Les indices de mouillage, représentés par les valeurs de l'angle thêta pris au point de raccordement d'une goutte de liquide avec la surface du matériau testé ont été mesurés en comparant le film de polycarbonate avant ou après traitement par plasma, et après traitement par plasma puis recouvrement par le polymère hydrophile.

On a utilisé comme matériau de départ un film de polycarbonate de 20 µm d'épaisseur, ayant une taille de pore de 30 nm et une densité de pores de 8 X 10⁹/cm².

Le traitement par plasma et le recouvrement par une couche de polymère ont été réalisés comme décrit à l'exemple 1.

Avant tout traitement, on a mesuré une valeur d'angle d'environ 74° pour le film de polycarbonate, ce qui reflète une hydrophobicité de la surface de ce film.

Après traitement par plasma d'argon, la valeur d'angle est d'environ 29°. La création de sites polaires à la surface du film de polycarbonate a donc fortement augmentée l'énergie de surface et le caractère hydrophile de ce film.

Après recouvrement du film de polycarbonate prétraité par plasma d'argon par une couche d'un polymère hydrophile, en l'occurrence la PVP, on peut mesurer une valeur d'angle d'environ 22°.

Ainsi, la couche de polymère hydrophile augmente significativement les propriétés d'hydrophilicité du film de polycarbonate.

On a donc montré que la surface d'une membrane semi-perméable conforme à l'invention avait des caractéristiques d'hydrophilicité significativement accrues par rapport au film de polycarbonate non traité.

De plus, la rétention du polymère hydrophile à la surface du film de polycarbonate grâce à la création de sites polaires permet un maintien des caractéristiques d'hydrophilicité pendant une longue période.

### EXEMPLE 3: Caractéristiques de perméabilité d'une membrane semi-perméable conforme à l'invention.

### a) Matériels et Méthodes

### - Mesure de la perméabilité au glucose, à l'insuline et aux immunoglobulines (IgG).

### 1. Test de perméabilité passive

La perméabilité au glucose, à l'insuline, aux immunoglobulines des membranes polycarbonate traitées ou non est évaluée au moyen d'une chambre de diffusion. Cette chambre est composée de deux compartiments de 3 ml chacun séparés par la membrane à tester d'une surface de 3,8 cm². Les tests sont réalisés à 37°C avec une concentration initiale en glucose de 4 g/l, en insuline humaine de 4 mUI/ml et en IGG bovine à la concentration de 1 mg/ml. Au temps O, 0.5, 1, 4, 8, 12 et 24 heures, des prélèvements sont effectués dans les deux compartiments pour la mesure du glucose selon une méthode enzymatique de l'insuline par RIA et des immunoglobulines par colorimétrie (Bradford).

### 2. Test de perméabilité active

La perméabilité active aux IgG est réalisée à température ambiante à l'aide d'une chambre d'ultrafiltration d'un volume de 12 ml équipée de la membrane à tester. La perméabilité aux immunoglobulines est évaluée sous une pression de 1 bar. La concentration en protéine est dosée dans l'ultrafiltrat après une heure par la méthode de Bradford.

### b) Résultats

La perméabilité d'une membrane semi-perméable réalisée à partir d'un film de polycarbonate ayant une taille de pore de 30 nanomètres et une densité de pore de 8.10⁹ pore/cm² commercialisée par la Société Wathman Group a été étudiée à 20°C pendant 1 heure, 8 heures et 24 heures.

Les résultats sont présentés dans le tableau 1 ci-après.

**TABLEAU 1**

| | | 20°C | | |
|---|---|---|---|---|
| Temps (heure) | 0 | 1 heure | 8 heures | 24 heures |
| Polycarbonate | 0 | 2% | 23% | 41% |
| Polycarbonate+ Plasma Argon | 0 | 4% | 48% | 50% |
| Polycarbonate + Plasma Argon | 0 | 15% | 49% | 52% |
| + PVP(1%) | | | | |

Les résultats du tableau 1 montrent que la membrane semi-perméable conforme à l'invention a des propriétés de perméabilité au glucose environ huit fois supérieures au film de polycarbonate après 1 heure, et environ 3 fois supérieures au film de polycarbonate après traitement par plasma d'argon durant la même période de temps.

Ces résultats montrent que la membrane semi-perméable de l'invention permet des échanges rapides de petites molécules de part et d'autre de la membrane.

La rapidité du flux des échanges des substances biologiquement actives d'intérêt de nature à permettre un apport rapide de l'organisme en substances biologiquement actives d'intérêt produites par un organe bio-artificiel doté d'une membrane semi-perméable conforme à l'invention.

La capacité du flux d'échange de petites molécules à travers la membrane semi-perméable de l'invention est encore significative au bout de 24 heures (voir tableau 1).

Les résultats de perméabilité au glucose mesurés à 37°C sont représentés dans le tableau 2 ci-après.

**TABLEAU 2**

| | | 37°C | | |
|---|---|---|---|---|
| Temps (heures) | 0 | 1 heure | 8 heures | 24 heures |
| Polycarbonate | 0 | 10% | 20% | 51% |
| Polycarbonate | 0 | 15% | 52% | 55% |
| + Plasma argon | | | | |
| + PVP (1%) | | | | |

Les résultats du tableau 2 montrent qu'à 37°C, le flux des échanges de petites molécules de part et d'autre d'une membrane semi-perméable conforme à l'invention est supérieur de 50% par rapport au film de polycarbonate poreux non traité, après 1 heure.

Des tests de perméabilité aux immunoglobulines (IgG) d'une membrane semi-perméable conforme à l'invention ont été réalisés à la température de 37°C.

Les résultats montrent qu'au bout de 8 heures de test, la perméabilité aux IgG est de l'ordre de 1 %.

L'ensemble des résultats ci-dessus montre que la membrane semi-perméable selon l'invention permet un passage rapide des petites molécules mais est imperméable aux molécules de poids moléculaire de plusieurs centaines de kilo Daltons telles que les IgG.

En conséquence, un organe bio-artificiel doté d'une membrane semi-perméable de l'invention permet une accessibilité rapide des substances biologiquement actives produites par cet organe bio-artificiel aux cibles de l'organisme hôte tout en restant inaccessible aux anticorps et aux cellules du système immunitaire de cet organisme hôte.

En outre, le passage rapide de petites molécules est de nature à permettre une grande accessibilité des différents nutriments nécessaires à une bonne viabilité des cellules contenues dans l'organe bio-artificiel ainsi que; le cas échéant, de molécules signal produites par l'organisme hôte et qui peuvent avoir un rôle dans la régulation de la production des substances biologiquement actives produites par l'organe bio-artificiel, comme par exemple le glucagon qui régule la synthèse d'insuline par les cellules des îlots de Langherans.

### EXEMPLE 4: Adsorption des protéines

### a) Matériels et Méthodes

Les supports sur lesquels les protéines ont été adsorbées consistaient d'une part en un film de polycarbonate de bisphénol A (PC), de 15 micromètres d'épaisseur, provenant de la firme GENERAL ELECTRIC (Lexan 8800) et, d'autre part, de membranes hydrophiles de 10 micromètres d'épaisseur, provenant de la S.A. WATHMAN (Louvain-la-Neuve, Belgique), présentant des pores de 400 nm de diamètre avec une densité de 6.10⁸ pores/cm². Certaines membranes avaient été traitées par plasma d'argon (50 W, 10 min) et ensuite trempées dans une solution de polyvinylpyrrolidone.

Les adsorptions ont été réalisées en incubant à 37°C pendant 2 heures les échantillons (disque de 13 mm de diamètre) dans 2 ml de solutions d'albumine bovine (bovine serum albumin, Sigma - St Louis MI) et d'insuline humaine (Novo Nordisk, Denmark) de concentration variable (0≤c≤10 mg/ml pour l'albumine et 0 ≤ c 10 µl/ml pour l'insuline). Les échantillons ont été ensuite rincés 3 fois avec du PBS (phosphate buffer saline, ajusté à un pH de 7.4) et 3 fois avec de l'eau ultrapure. Ils ont été ensuite séché sous flux d'azote et stockés avant analyse dans un dessiccateur contenant du P₂O₅.

### b) Résultats

L'adsorption d'albumine sur une membrane semi-perméable conforme à l'invention a été étudiée par corrélation entre les résultats d'analyse de spectroscopie XPS (« X-ray photoelectron spectroscopy ») et Tof-Sims (« Time of flight secondary ion mass spectroscopy ») selon la technique décrite par Rouxhet L. et Bertrand P. (« Secondary ion mass spectrometry, Sims XII », eds. A. Benninghoven, Bertrand P. H.-N. Migeon et W. Werner (Editors), Elsevier Science B.V. Publ., 2000, 907-910).

Les résultats montrent que l'adsorption d'albumine ou d'insuline atteint rapidement un plateau pour des concentrations en ces protéines de l'ordre du milligramme par millilitre, indiquant que ces protéines couvrent la surface de la membrane semi-perméable sous la forme d'une mono-couche ou encore d'une mono-couche partielle.

Les résultats obtenus montrent que la membrane semi-perméable selon l'invention, du fait de la présence de la couche d'au moins un polymère hydrophile, a une capacité réduite d'adsorption des protéines à sa surface, ce qui est de nature à maintenir ces capacités de perméabilité pendant une longue période de temps.

### EXEMPLE 5: Analyse des sites polaires créés à la surface du film de polycarbonate après traitement par plasma d'argon.

### a) Matériels et Méthodes

L'analyse des sites polaires à la surface des différentes membranes étudiées a été réalisée par corrélation entre les résultats d'analyse de spectroscopie XPS et Tof-Sims, comme pour l'exemple 4.

### b) Résultats

Un film de polycarbonate commercialisé par la Société Whatman Group et ayant une épaisseur de 16 µm, une taille de pore de 30 nanomètres et une densité de pores de 2.10⁹ pores/cm² a été analysé par XPS et TOF-SIMS avant et après traitement par plasma d'argon.

Avant traitement par plasma d'argon, l'analyse ToF-SIMS a montré les pics caractéristiques du polycarbonate (en mode négatif à m/z= 60, 93, 117, 133, 149, 211, 227, 255 et en mode positif à m/z = 135, 213, 329, 71, 91, 105, 128, 141, 152, 165, 178, 193). La présence de PVP est également détectée au moyen de ses pics caractéristiques (en mode positif à m/z = 41, 69, 86, 98, 112, 124, 138, 207, 233 et en mode négatif à m/z = 26 (CN), 42 (CNO), 84, 108). Ceci résulte d'un traitement initial par trempage réalisé par le fabricant de la membrane.

Après traitement par plasma d'argon, les pics suivants ont été observés :
- en mode positif (m/z): 31 (CH₃O), 43(C₂H₃O), 55(C₃H₃O), 59(C₃H₇O), 18 (NH₄⁺) et 46(C₂H₈N⁺); et
- en mode négatif (m/z): 16 (O), 17(OH), 41(C₂OH), 117(C₈H₅O).

Les résultats ci-dessus montrent que le traitement par plasma d'argon a permis la création de nombreux sites polaires à la surface du film de polycarbonate qui permettent, au travers de la création de liaisons faibles, une bonne adhésion de la couche d'au moins un polymère hydrophile et son maintien durable à la surface du film de polycarbonate.

### EXEMPLE 6 - Pancréas bio-artificiel comprenant des membranes semi-perméables conformes à l'invention.

Un pancréas bio-artificiel comprenant plusieurs membranes semi-perméables conformes à l'invention a été construit comme illustré à la figure 1.

La structure du pancréas bio-artificiel comprenant un support de silicone est commercialisée par la Société STATICE.

Un pancréas bio-artificiel contenant 20.000 îlots de Langherans pancréatique a été fabriqué pour implantation chez le porc.

Le pancréas bio-artificiel est composé de 20 chambres d'encapsulation comprenant chacune trois membranes semi-perméables de l'invention, chacune des chambres d'encapsulation étant solidaire d'un support flexible en silicone.

Le pancréas bio-artificiel a été implanté dans la cavité péritonéale de porcs miniatures sous anesthésie au moyen d'une laparotomie.

Après un mois d'implantation, le pancréas bio-artificiel a été retiré afin d'analyser sa résistance mécanique ainsi que l'état de sa surface par microscopie électronique à balayage.

Les résultats montrent que le pancréas bio-artificiel n'a pas subi d'altération mécanique: aucune chambre d'encapsulation ne s'est désolidarisée du système ou n'a été ouverte sous l'effet des contraintes mécaniques.

En outre, le résultat de l'analyse des clichés de microscopie photonique, après lavage des membranes et coloration histologique, a montré qu'aucune cellule n'avait adhéré sur l'implant, qu'il s'agisse du support en silicone ou de la membrane semi-perméable de l'invention.

Par comparaison, un support en fibres de polyester implanté parallèlement chez le porc miniature était couvert de fibroblastes et de dépôts de fibrine.

## Revendications

1. Membrane semi-perméable pour chambre d'encapsulation de cellules produisant au moins une substance biologiquement active d'intérêt, **caractérisée en ce que** ladite membrane semi-parméable consiste en un film biocompatible de polycarbonate poreux modifié en surface par création de sites polaires et recouvert sans greffage chimique par une couche d'au moins un polymère hydrophile.

2. Membrane selon la revendication 1, **caractérisée en ce qu'**elle possède un seuil de coupure compris entre 10 000 et 50 000 Daltons, de préférence entre 10 000 et 30 000 Daltons.

3. Membrane selon l'une des revendications 1 et 2, **caractérisée en ce que** la taille des pores du film de polycarbonate est comprise entre 5 et 30 nanomètres, de préférence entre 5 et 15 nanomètres.

4. Membrane selon l'une des revendications 1 à 3, **caractérisée en ce qu'**elle possède un densité de pores comprise entre 10⁹ et 10¹¹ pores/cm².

5. Membrane selon l'une des revendications 1 à 4, **caractérisée en ce que** la couche d'au moins un polymère hydrophile possède une épaisseur comprise entre 10 et 100 nanomètres.

6. Membrane selon la revendication 1, **caractérisée en ce qu'**elle possède une épaisseur comprise entre 5 µm et 25 µm.

7. Membrane selon l'une des revendications 1 à 6, **caractérisée en ce que** le polymère hydrophile est choisi parmi les celluloses et leurs dérivés, les polyacrylamides et leurs copolymères, la polyvinylpyrrolidone (PVP) et ses copolymères, les copolymères de l'acétate de vinyle et de l'alcool vinylique, les polyéthylène glycols, les propylène glycols, les propylène glycols, les poly(méth)acrylates hydrophiles, les polyosides et les chitosans.

8. Chambre d'encapsulation de cellules produisant au moins une substance biologiquement active d'intérêt **caractérisée en ce qu'**elle comprend au moins une membrane selon l'une des revendications 1 à 7.

9. Chambre d'encapsulation selon la revendication 8, **caractérisée en ce qu'**elle comprend deux membranes, respectivement inférieures (2) et supérieures (1), selon l'une des revendications 1 à 6 dont les bords sont solidaires d'un support (4), les deux membranes délimitant un espace susceptible de contenir les cellules produisant au moins une substance biologiquement active d'intérêt.

10. Chambre d'encapsulation selon la revendication 9, **caractérisée en ce que** chacune des membranes est de forme circulaire.

11. Chambre d'encapsulation selon l'une des revendications 8 à 10, **caractérisée en ce qu'**elle contient des cellules produisant au moins une substance biologiquement active d'intérêt.

12. Chambre d'encapsulation selon la revendication 11, **caractérisée en ce que** les cellules sont choisies parmi les cellules hépatiques et les cellules des îlots de Langherans.

13. Chambre d'encapsulation selon la revendication 11, **caractérisée en ce que** les cellules sont transformées par au moins un acide nucléique permettant l'expression d'une substance biologiquement active d'intérêt.

14. Chambre d'encapsulation selon la revendication 11, **caractérisée en ce que** la substance biologiquement active d'intérêt est choisie parmi l'insuline, les cytokines, les hormones peptidiques, l'hormone de croissance et la calcitonine.

15. Organe bio-artificiel **caractérisé en ce qu'**il comprend une chambre d'encapsulation ou une pluralité de chambres d'encapsulation selon l'une quelconque des revendications 8 à 14.

16. Organe bio-artificiel selon la revendication 15, **caractérisé en ce qu'**il s'agit d'un pancréas artificiel contenant des cellules des îlots de Langherans.

17. Organe bio-artificiel selon la revendication 14, **caractérisé en ce qu'**il s'agit d'un pancréas artificiel dans lequel la chambre d'encapsulation ou la pluralité de chambres d'encapsulation comprend des cellules des îlots de Langherans, les cellules étant de préférence inclues dans une matrice de collagène.

18. Procédé d'obtention d'une membrane semi-perméable pour chambre d'encapsulation de cellules produisant au moins une substance biologiquement active d'intérêt, **caractérisé en ce qu'**il comprend les étapes suivantes :
a) création de sites polaires à la surface d'un film biocompatible de polycarbonate poreux ;
b) recouvrement du film de polycarbonate ainsi traité par une couche d'au moins un polymère hydrophile sans greffage chimique ; et
c) séchage.

19. Procédé selon la revendication 18, **caractérisé en ce que** l'étape a) est réalisée par un traitement par plasma, par décharge couronne ou encore par décharge électromagnétique à pression atmosphérique ou sous vide.

20. Procédé selon l'une des revendications 18 ou 19, **caractérisé en ce que** dans l'étape b), le polymère hydrophile est choisi parmi les celluloses et leurs dérivés, les polyacrylamides et leurs copolymères, la polyvinylpyrrolidone (PVP) et ses copolymères, les copolymères de l'acétate de vinyle et de l'alcool vinylique, les polyéthylène glycols, les propylène glycols, les propylène glycols, les poly(méth)acrylates hydrophiles, les polyosides et les chitosans.

21. Procédé selon l'une des revendications 18 à 20, **caractérisé en ce que** l'étape b) est réalisée avec une solution aqueuse d'au moins un polymère hydrophile qui possède une viscosité comprise entre 1 et 10 centipoises.

## Claims

1. A semi-permeable membrane for a cell encapsulation chamber producing at least one biologically active substance of interest, **characterized in that** the said semi-permeable membrane consists of a porous polycarbonate biocompatible film modified at the surface through creation of polar sites and coated, without chemical grafting, with a layer of at least one hydrophilic polymer.

2. A membrane according to claim 1, **characterized in that** it has a cutoff threshold ranging from 10,000 to 50,000 Daltons, preferably from 10,000 to 30,000 Daltons.

3. A membrane according to any one of claims 1 and 2, **characterized in that** the pore size of the polycarbonate film ranges from 5 to 30 nanometers, preferably from 5 to 15 nanometers.

4. A membrane according to any one of claims 1 to 3, **characterized in that** it has a pore density ranging from 10⁹ and 10¹¹ pores/cm².

5. A membrane according to any one of claims 1 to 4, **characterized in that** the layer of at least one hydrophilic polymer has a thickness ranging from 10 to 100 nanometers.

6. A membrane according to claim 1, **characterized in that** it has a thickness ranging from 5 to 25 µm.

7. A membrane according to any one of claims 1 to 6, **characterized in that** the hydrophilic polymer is selected amongst celluloses and the derivatives thereof, polyacrylamides and the copolymers thereof, polyvinylpyrrolidone (PVP) and the copolymers thereof, vinyl acetate and vinyl alcohol copolymers, polyethylene glycols, propylene glycols, hydrophilic poly(meth)acrylates, polyosides and chitosans.

8. A cell encapsulation chamber producing at least one biologically active substance of interest, **characterized in that** it comprises at least one membrane according to any one of claims 1 to 7.

9. An encapsulation chamber according to claim 8, **characterized in that** it comprises two membranes, respectively a bottom (2) and at top (1) membrane, according to any one of claims 1 to 6, the edges of which are integral with a support (4), both membranes delimiting a space able to contain the cells producing at least one biologically active substance of interest.

10. An encapsulation chamber according to claim 9, **characterized in that** each of the membranes is circularly shaped.

11. An encapsulation chamber according to any one of claims 1 to 10, **characterized in that** it contains cells producing at least one biologically active substance of interest.

12. An encapsulation chamber according to claim 11, **characterized in that** the cells are selected amongst hepatic cells and islets of Langherans cells.

13. An encapsulation chamber according to claim 11, **characterized in that** the cells are transformed by at least one nucleic acid allowing the expression of a biologically active substance of interest.

14. An encapsulation chamber according to claim 11, **characterized in that** the biologically active substance of interest is selected amongst insulin, cytokines, peptide hormones, growth hormone and calcitonin.

15. A bioartificial organ, **characterized in that** it comprises an encapsulation chamber or a plurality of encapsulation chambers according to any one of claims 8 to 14.

16. A bioartificial organ according to claim 15, **characterized in that** it is an artificial pancreas containing islets of Langherans cells,

17. A bioartificial organ according to claim 14, **characterized in that** it is an artificial organ in which the encapsulation chamber or the plurality of encapsulation chambers comprises islets of Langherans cells, the cells being preferably included within a collagen matrix.

18. A method for obtaining a semi-permeable membrane for a cell encapsulation chamber producing at least a biologically active substance of interest, **characterized in that** it comprises the following steps of:
a) creating polar sites at the surface of a porous polycarbonate biocompatible film;
b) coating the thus treated polycarbonate film with a layer of at least one hydrophilic polymer, without a chemincal grafting ; and
c) drying.

19. A method according to claim 18, **characterized in that** step a) is conducted through a plasma treatment, through corona discharge as well as through electromagnetic discharge at atmospheric pressure or under vacuum.

20. A method according to any of claims 18 or 19, **characterized in that** in step b), the hydrophilic polymer is selected amongst celluloses and the derivates thereof, polyacrylamides and the copolymers thereof, polyvinylpyrrolidone (PVP) and the copolymers thereof, vinyl acetate and vinyl alcohol copolymers, polyethylene glycols, propylene glycols, hydrophilic poly(meth)acrylates, polyosides and chitosans.

21. A method according to any one of claims 18 to 20, **characterized in that** step b) is conducted with an aqueous solution of at least one hydrophilic polymer having a viscosity ranging from 1 to 10 centipoises.

## Patentansprüche

1. Semipermeable Membran für eine Kammer zur Einkapselung von Zellen, die mindestens eine biologisch aktive Substanz von Interesse produzieren, **dadurch gekennzeichnet, dass** die genannte semipermeable Membran aus einem biokompatiblen, porösen Polycarbonatfilm besteht, der durch Bildung von polaren Stellen oberflächenmodifiziert und ohne chemische Pfropfung mit einer Schicht von mindestens einem hydrophilen Polymer bedeckt ist.

2. Membran nach Anspruch 1, **dadurch gekennzeichnet, dass** sie eine Ausschlussgrenze zwischen 10 000 und 50 000 Dalton und vorzugsweise zwischen 10 000 und 30 000 Dalton aufweist.

3. Membran nach einem der Ansprüche 1 und 2, **dadurch gekennzeichnet, dass** die Größe der Poren des Polycarbonatfilms zwischen 5 und 30 Nanometern und vorzugsweise zwischen 5 und 15 Nanometern liegt.

4. Membran nach einem der Ansprüche 1 bis 3, **dadurch gekennzeichnet, dass** sie eine Porendichte zwischen 10⁹ und 10¹¹ Poren/cm² aufweist.

5. Membran nach einem der Ansprüche 1 bis 4, **dadurch gekennzeichnet, dass** die Schicht von mindestens einem hydrophilen Polymer eine Dicke zwischen 10 und 100 Nanometern aufweist.

6. Membran nach Anspruch 1, **dadurch gekennzeichnet, dass** sie zwischen 5 *µ*m und 25 µm dick ist.

7. Membran nach einem der Ansprüche 1 bis 6, **dadurch gekennzeichnet, dass** das hydrophile Polymer unter Cellulosen und deren Derivaten, Polyacrylamiden und deren Copolymeren, Polyvinylpyrrolidon (PVP) und dessen Copolymeren, Vinylacetat- und Vinylalkohol-Copolymeren, Polyethylenglykolen, Propylenglykolen, hydrophilen Poly(meth)acrylaten, Polyosiden und Chitosanen ausgewählt wird.

8. Kammer für die Einkapselung von Zellen, die mindestens eine biologisch aktive Substanz von Interesse produzieren, **dadurch gekennzeichnet, dass** sie mindestens eine Membran nach einem der Ansprüche 1 bis 7 enthält.

9. Einkapselungskammer nach Anspruch 8, **dadurch gekennzeichnet, dass** sie jeweils zwei untere (2) und obere (1) Membranen nach einem der Ansprüche 1 bis 6 enthält, deren Ränder mit einem Träger (4) fest verbunden sind, wobei die beiden Membranen einen Raum begrenzen, in dem Zellen enthalten sein können, die mindestens eine biologisch aktive Substanz von Interesse produzieren.

10. Einkapselungskammer nach Anspruch 9, **dadurch gekennzeichnet, dass** jede der Membranen kreisförmig ist.

11. Einkapselungskammer nach einem der Ansprüche 8 bis 10, **dadurch gekennzeichnet, dass** sie Zellen enthält, die mindestens eine biologisch aktive Substanz von Interesse produzieren.

12. Einkapselungskammer nach Anspruch 11, **dadurch gekennzeichnet, dass** die Zellen aus hepatischen Zellen und Langerhansinseln ausgewählt wurden.

13. Einkapselungskammer nach Anspruch 11, **dadurch gekennzeichnet, dass** die Zellen durch mindestens eine Nukleinsäure umgewandelt sind, was die Expression einer biologisch aktiven Substanz von Interesse ermöglicht.

14. Einkapselungskammer nach Anspruch 11, **dadurch gekennzeichnet, dass** die biologisch aktive Substanz von Interesse unter Insulin, Cytokinen, Peptidhormonen, Wachstumshormonen und Calcitonin ausgewählt wird.

15. Bioartifizielles Organ, **dadurch gekennzeichnet, dass** es eine Einkapselungskammer oder eine Mehrzahl von Einkapselungskammern nach einem der Ansprüche 8 bis 14 enthält.

16. Bioartifizielles Organ nach Patentenanspruch 15, **dadurch gekennzeichnet, dass** es sich um einen künstlichen Pankreas handelt, der Zellen der Langerhansinseln enthält.

17. Bioartifizielles Organ nach Anspruch 14, **dadurch gekennzeichnet, dass** es sich um einen künstlichen Pankreas handelt, in welchem die Einkapselungskammer oder die Mehrzahl der Einkapselungskammern Zellen der Langerhansinseln enthalten, wobei die Zellen vorzugsweise in einer Kollagenmatrix eingeschlossen sind.

18. Verfahren zum Erhalt einer semipermeablen Membran für eine Kammer zur Einkapselung von Zellen, die mindestens eine biologisch aktive Substanz von Interesse produzieren, **dadurch gekennzeichnet, dass** es die folgenden Schritte umfasst:
a) Bildung von polaren Stellen an der Oberfläche eines biokompatiblen, porösen Polycarbonatfilms,
b) Überzug des so behandelten Polycarbonatfilms durch eine Schicht von mindestens einem hydrophilen Polymer ohne chemische Pfropfung und
c) Trocknung.

19. Verfahren nach Anspruch 18, **dadurch gekennzeichnet, dass** der Schritt a) durch Plasmabehandlung, durch Koronaentladung oder durch elektromagnetische Entladung bei atmosphärischem Druck oder im Vakuum erfolgt.

20. Verfahren nach einem der Ansprüche 18 oder 19, **dadurch gekennzeichnet, dass** in Schritt b) das hydrophile Polymer unter Cellulosen und deren Derivaten, Polyacrylamiden und deren Copolymeren, Polyvinylpyrrolidon (PVP) und dessen Copolymeren, Vinylacetat- und Vinylalkohol-Copolymeren, Polyethylenglykolen, Propylenglykolen, hydrophilen Poly(meth)acrylaten, Polyosiden und Chitosanen ausgewählt wird.

21. Verfahren nach einem der Ansprüche 18 bis 20, **dadurch gekennzeichnet, dass** der Schritt b) mit einer wässrigen Lösung mindestens eines hydrophilen Polymers mit einer Viskosität zwischen 1 und 10 Centipoise erfolgt.
